(19) Europäisches Patentamt
European Patent Office
Office européen des brevets

(11) Publication number: **0 275 559 B1**

(12) # EUROPEAN PATENT SPECIFICATION

(45) Date of publication of patent specification: **26.06.91**

(51) Int. Cl.$^5$: **C07F 15/00, A61K 31/28**

(21) Application number: **87119339.7**

(22) Date of filing: **29.12.87**

The file contains technical information submitted after the application was filed and not included in this specification

(54) **Platinum cytostatic agent.**

(30) Priority: **29.12.86 CS 10074/86**

(43) Date of publication of application:
**27.07.88 Bulletin 88/30**

(45) Publication of the grant of the patent:
**26.06.91 Bulletin 91/26**

(84) Designated Contracting States:
**AT BE CH DE ES FR GB IT LI LU NL SE**

(56) References cited:
**EP-A- 0 181 166**
**AT-B- 375 326**
**US-A- 4 140 707**

(73) Proprietor: **LACHEMA, n.p.**
**Karásek 28**
**Brno-Reckovice(CS)**

(72) Inventor: **Kiss, Frantisek, Dipl.-Ing.**
**Bednárová 20**
**Brno(CS)**
Inventor: **Závodná, Ivanka RNDr**
**Hoblikova 8**
**Brno(CS)**
Inventor: **Novotny, Jiri, Dipl.-Ing.**
**Fleischnerova 9**
**Brno(CS)**
Inventor: **Hájek, Eduard, RNDr**
**Muzikova 13**
**Brno(CS)**
Inventor: **Bohuminská, Monika, Dipl.-Ing.**
**Koutného 9**
**Brno(CS)**
Inventor: **Marciková, Ludmila**
**Lastuvkova 13**
**Brno(CS)**
Inventor: **Uhrová, Zdenka**
**Noskova 7**
**Brno(CS)**
Inventor: **Borák, Jindrich, Dipl.-Ing.**
**Nám. SNP 21**
**Brno(CS)**
Inventor: **Salamoun, Jaroslav, Dipl.-Ing.**
**Hlavni 245**
**Dolni Loucky(CS)**

(74) Representative: **Patentanwälte Beetz sen. - Beetz jun. Timpe - Siegfried - Schmitt-Fumian**
**Steinsdorfstrasse 10**
**W-8000 München 22(DE)**

**EP 0 275 559 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a micro-crystalline cytostatic agent on the basis of cis-diammine-1,1-cyclobutanedicarboxylate platinum complex of the general formula I,

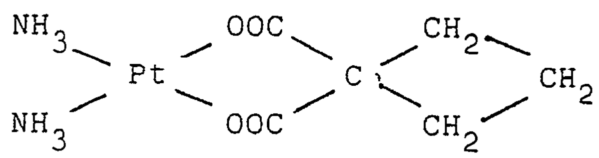
(I),

which has anti-neoplastic activity, a method of preparing this cytostatic agent and pharmaceutical compositions comprising it as active ingredient.

The complex I belongs to the first platinum coordination compounds which have been synthetized not long after the discovery of the cytostatic activity of cis-platinum (cis-DDP = cis-diamminedichloroplatinum complex) and analogues thereof characterised by the cis-configuration of amino-(A) and acidoligands (X) of the general formula

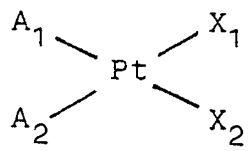

Extensive research work has been focused to develop or find easily soluble, hydrolytically stable and active complexes with high anti-neoplastic activity and minimal unwanted side-effects, particularly low toxicity, viz. complexes having a high therapeutical index.

The complex I has been synthetized for the first time together with other malonate-platinum(II) compounds in 1972 (US-A-4 140 707), and since that time the advantages of this cytostatic agent, as compared with other tested complexes, have been confirmed by various experimental and clinical studies on the basis of which complex I was allowed in 1986 to be applied to the therapy of certain types of malign diseases in several countries.

The known process of synthesis of complex I according to Dhara (Indian J. Chem. 8 (1970) 193) can be described by the following reaction scheme:

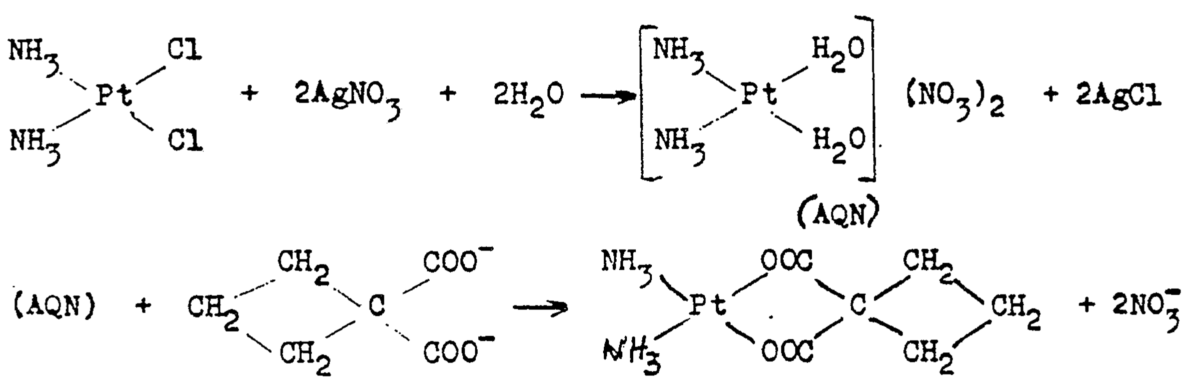


The two reaction steps are carried out at an elevated temperature, and complex I is isolated after cooling the reaction mixture. The quality of recrystallized products is proved by a fair agreement between theoretical and experimentally ascertained elementary compositions (see US-A-4 140 707).

The process as explained above, although it is most commonly utilized, has many disadvantages. In the first stage of synthesis, it is necessary to keep the ratios of the starting components entering the reaction very exactly, and, optionally, to remove an excess of $Ag^+$ by adding $Cl^-$, which requires repeated

centrifugation or ultrafiltration.

Colloidal silver chloride as well as aqua-nitrate-platinum complex (AQN) are very light-sensitive substances; the former is apt to decompose to colloidal silver whose both catalytic and biological activity is unwanted in this case while the latter (AQN), in accordance with recent NMR examination results, may produce highly toxic oligomers which after all are ineffective from the viewpoint of antineoplastic therapy (Inorg. Chem. 16 (1970) 1525 and 1192; J. Am. Chem. Soc. 99 (1977) 777).

These facts influence the course of subsidiary reactions in the second stage of synthesis, whereby unsoluble complexes have a very similar elementary composition are formed which occur in the product, viz. complex I. Such by-products have to be removed from the reaction mixture as early as in the hot state, e.g. by filtration, and further on by recrystallization of the crude complex I.

Detailed analytical investigations of the product by sensitive chromatographic methods (TLC, HPLC) and spectrometrical methods (NMR, AAS) proved that the thus synthetized complex I attains a 97 to 98 percent purity in quite extraordinary cases only, and that it always contains about $10^{-3}$ % of silver, i.e. an amount which is at the limit of general pharmacopoiea standards; the Czechoslovak pharmacopoiea 3 allows an Ag content in therapeutical agents of $\leqq 10^{-3}$ %.

There is no indication in scientific publications or patent literature about the possibility of preparing and about properties of a micro-crystalline complex I. Regarding platinum based cytostatic agents there is solely known a process for preparing micronized cis-platinum by precipitating it from dimethylformamide solutions by means of hydrochloric acid.

The physical properties of complex I preparations obtained according to the hitherto known methods are not advantageous from the viewpoint of further processing it into galenic forms suitable for administration. The substance is polycrystalline, has a coarse grain size distribution within a broad range of 50 to 2000 µm, and the particles are soluble in water at a slow rate only. From the technological viewpoint, such a product is not suitable to be used for preparing solutions designed for lyophilization in order to obtain micro-crystalline galenic forms and is quite unsatisfactory for preparing sterile pulverulent mixtures for injection, i.e. the so-called dry injections.

It is an object of the present invention to eliminate the drawbacks of prior art as hereinabove set forth and to provide an improved platinum cytostatic agent on the basis of cis-diammine-1,1-cyclobutanedicarboxylate platinum(II) of the general formula I (see claim 1), a method for producing this cytostatic agent, and corresponding pharmaceutical compositions.

This object is achieved according to claims 1, 3 and 14. The dependent claims relate to preferred embodiments.

According to the invention, the cytostatic agent on the basis of cis-diammine-1,1-cyclobutanedicarboxylate platinum(II) complex of the general formula I

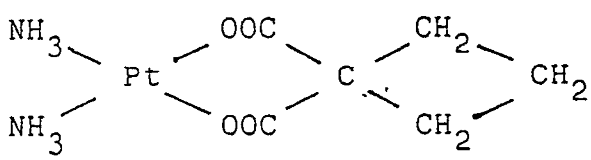

(I),

is characterised in that it comprises at least 60 % of particles having a particle size of from zero to 25 µm, at least 95 % of particles having a particle size of from zero to 50 µm and at least 99 % of particles having a particle size of from zero to 75 µm, and having a purity of at least 98 % and a silver content not exceeding $10^{-5}$%.

The method according to the invention for preparing the cytostactic agent on the basis of the platinum complex I comprises the following essential steps:

(A) agitating and/or applying a reaction mixture having a pH of 4 to 11 and comprising

- 1 part by mass of a cis-diamminedihalogen platinum(II) complex of general formula II,

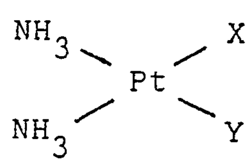

(II),.

wherein X and Y are Cl, Br or I

and/or of products of hydrolysis of complex of formula II,
- 1 to 5 parts by mass of 1,1-cyclobutanedicarboxylic acid and/or salts thereof and/or esters thereof,
- 3 to 30 parts by mass of water, optionally containing a water-miscible organic solvent in an amount of 1 to 10 parts by mass, such as ethanol, isopropanol and/or glycerol,
- and optionally 0,01 to 2 parts by mass of a sorbent

at a temperature of 20 to 100 °C,

(B) optionally purifying the mixture by addition of 0,01 to 0,5 parts by mass of a sorbent,

(C) filtering the mixture,

(D) cooling down the product to a temperature of -5 to +50 °C, preferably at a rate of 2 to 25 °C/min, and preferably under agitation and/or exposure to sound waves, particularly of a frequency of 15 to 20 kHz, and

(E) isolating the cytostatic agent and optionally washing, drying and sorting and/or classifying.

If necessary, according to the invention the complex I is treated by dissolving 1 part by mass thereof in 5 to 25 parts by mass of water containing, optionally, 0,001 to 0,1 parts by mass of 1,1-cyclobutanedicarboxylic acid and/or a salt thereof at a temperature of 70 to 95 °C, optionally, with addition of 0,002 to 0,5 parts by mass of sorbent, filtering and cooling the solution to a temperature of -5 to 50 °C at a rate of 2 to 25 °C/min under agitation and, preferably, exposure to sound waves, e.g. of a frequency of 15 to 20 kHz, isolating the micro-crystalline product, washing it, and, optionally, drying and sorting or classifying.

The sorbent which may be used in the treatment of complex I is preferably activated carbon and/or an ion-exchanger.

The complex I, after having been isolated, is preferably washed with water and/or dimethylformamide and/or dimethylacetamide.

The pharmacological compositions according to the invention are characterised in that they comprise the cytostatic agent as defined above together with pharmacologically acceptable adjuvants and/or carriers and/or excipients.

According to a preferred embodiment, the pharmacological compositions consist of a sterile, dry micro-crystalline powder of the cytostatic agent on the basis of complex I and saccharides and/or organic acids and/or hydroxy acids and/or salts thereof and/or alkali metal and/or alkaline earth metal chlorides.

The pharmacological compositions comprise preferably 1 part by mass of the cytostatic agent as active ingredient and 0,1 to 10 parts by mass of adjuvants, carriers and/or excipients, particularly of lactose and/or glucose and/or saccharose and/or citric acid and/or ascorbic acid and/or salts thereof, and/or sodium chloride, potassium chloride, magnesium chloride and/or calcium chloride.

Injectable solutions consist preferably of an aqueous solution of the above component mixture comprising 1 part by mass of active ingredient and 0,1 to 10 parts by mass of additives, in 100 to 2000 parts by mass of water for injection of pH 2 to 7.

These injectable solutions can be made up immediately before use.

The platinum cytostatic agent on the basis of the micro-crystalline complex I has many advantageous properties. It is very easily soluble in water, aqueous glucose solutions, dextrose solutions or Ringer's solution. Dissolving a usual therapeutical dosis of 800 mg of sterile micro-crystalline complex I takes less than 3 minutes, i.e., the product according to the invention yields a dissolution rate approximately corresponding to that of the hitherto applied lyophilized injection product prepared by a relatively complex and technologically complicated process, which is rather time- and power-consuming.

The specific micro-crystalline form of complex I is the prerequisite for preparing therapeutical forms of this cytostatic agent, e.g. of dry preparations for injection. The relatively narrow particle size distribution of 5 to 50 $\mu$m is advantageous not only from the technological viewpoint but also in view of the dissolution rate before administration, since the physical properties of such a pulverulent material allow a reproducible dosage thereof for producing pharmaceutical dosage units which can be provided in suitable wrappings.

A further adjustment of the loose micro-crystalline cytostatic agent is possible by addition of pharmacologically admissible adjuvants which may contribute to a higher complex stability or to the attainment of physiological parameters of a ready-for-use solution made up before use which is designed for administration to patients.

The dry therapeutical form of the cytostatic agent is prepared either under completely aseptic conditions including the preparation of pharmacologically admissible adjuvants having an adequate purity and granularity, or under non-aseptic conditions with final sterilization of the resulting galenic form, e.g. by ionizing radiation.

The granulometric composition of the platinum cytostatic agent according to the present invention as well as its other properties are also advantageous for preparing other galenic forms, e.g. liquid or lyophilized preparations on the basis of complex I, optionally together with additives.

An extraordinarily important property of the cytostatic agent according to the invention is its high purity which, when tested by chromatographic methods (TLC and HPLC), attains values of 98 to 99,8 %, calculated on the basis of the water-free substance.

The silver content of the product is practically below the detection limit when examined by the flameless atomic absorption spectrophotometry, but always at a level of $10^{-5}$ %, i.e. two digits less than in specimens of complex I prepared by one of the heretofore known methods.

It is to be noted that the biological activity of the cytostatic agent, especially its anti-neoplastic activity or its therapeutical index, respectively (Neoplasma 31 (1948) 641-647), and its low toxicity, especially nephrotoxicity and gastrointestinal toxicity with experimental animals ($14^{th}$ International Cancer Congress, Budapest, 1986, Abstr. No. 2585, 4553, 4561, 4569), confirmed also with man during the $1^{st}$ and $2^{nd}$ clinical test (ibid., Abstr. No. 1746), correspond to the high purity thereof.

The advantages of the process for preparing the cytostatic agent according to the present invention reside not only in the above described product properties but also in the relatively low consumption of raw materials and the easier preparation as well as in the hygienic production conditions.

The synthesis of the complex I takes place in one single step without presence of silver nitrate or another silver compound, which makes the platinum recovery and recirculation easily controllable.

The yield of the reaction of cis-diamminedihalogen platinum(II) complex in step A is not, for certain ratios of reactants, too sensitive to a superstoichiometric among of the source of 1,1-cyclobutanedicarboxylate ligand, which makes it possible to use complex II for the synthesis not only in the form of a powder having defined properties but also in the form of solutions, suspensions, wet cakes or mixtures of hydrolysis products, without necessity to know exactly the exact content or composition of individual equilibrium components of complex II.

The use of sound wave energy, especially of ultrasonic waves, for agitating the heterogeneous reaction mixture as well as for obtaining positive values of both chemical and physical balance, is particularly advantageous with reaction systems of this type, wherein very rare biologically active substances are synthetized in relatively small reaction volumes while applying easily available ultra-sound sources.

The process according to the invention may also advantageously be applied for purifying complex I synthetized in a different way, such as e.g. to reduce the silver content of aquacomplexes, and to prepare complex I in the micronized form.

The invention will further be explained with reference to examples.

**Example1**

200 g of 1,1-cyclobutanedicarboxylic acid were dissolved in 500 ml of water, and, under agitation and cooling, the pH of the solution was adjusted to 5,9 by adding 710 g of 15 % sodium hydroxide solution. The solution was heated in a water bath to 88 °C with agitation, and 100 g of cis-diamminedichloroplatinum(II) complex suspended in 170 g of water were added thereto. At this temperature the reaction mixture was agitated 15 min whereupon after adding 1 g of activated carbon it was filtered through an activated carbon layer and a diaphragm of 0,2 µm porosity.

Under agitation and exposure to sound waves of a frequency of 20 kHz, the filtrate was cooled within 10 min to 23 °C. The crystallized product was then isolated by filtering the obtained suspension, successively washed with 80 g of water, dimethylformamide and again with water, sucked off and dried in a vacuum drier at a temperature of up to 50 °C.

There were obtained 73 g of a micro-crystalline product of complex I having a chromatographic purity of 98,5 % and consisting of 90 % of particles of up to 25 µm and 99 % of particles of up to 50 µm particle size.

50 mg of the substance when shaken in 10 ml of water were dissolved within 1 min.

**Example2**

a) 20 g of cis-$[(NH_3)_2PtBr_2]$ were suspended in 400 g of an aqueous disodium cyclobutanedicarboxylate solution (pH 6,5) containing (by calculation) 14,9 g of 1,1-cyclobutanedicarboxylic acid, 40 g of glycerol, 20 g of an anion-exchange resin in OH-cycle and 2 g of activated carbon. The reaction mixture was agitated 3 h at 50 °C whereupon it was filtered through a diaphragm (0,2 µm porosity), and the clear solution was cooled in an ultrasonic field under agitation to 20 °C. The crystallized product was isolated by filtering and successively washed with 30 ml of water, dimethylformamide and again with water.

b) 10 g of the thus prepared complex I were dissolved, under agitation and exposure to sound waves of a frequency of 20 kHz, in 120 g of water containing 1 g of 1,1-cyclobutanedicarboxylic acid, at 80 °C.

The solution was subjected to ultrafiltration and, under intensive agitation in an ultrasonic field, cooled within 5 min to 10 °C. The complex I was then isolated by filtration, washed with water and sucked off. A suspension of complex I in acetone was screened (width of mesh 40 µm); the finer fraction was isolated by filtering, sucked off and dried.

There were obtained 6 g of product having a chromatographic purity of 99,3 % and containing 99 % of particles smaller than 40 µm. 50 mg of the substance were dissolved in 10 ml of water within 30 s.

**Example3**

20 g of cis-diamminedichloroplatinum(II) complex were dissolved in 150 g of water (pH 7,5) and added, at 45 °C, with 15,6 g of 1,1-cyclobutanedicarboxylic acid ethyl ester and 10 g of an anion-exchange resin in OH-cycle whereupon the reaction mixture was agitated, while exposed to ultrasonic waves of a frequency of 50 kHz for 1,5 h. Then 0,5 g of activated carbon was added, and the mixture was subjected to ultrafiltration. Under agitation and exposure to ultra-sound of a frequency of 20 kHz, the solution was cooled within 2 min to 20 °C, and the separated complex I was isolated by filtration, washed with water and dimethylformamide and purified as described in example 2b.

There were obtained 6 g of micro-crystalline complex I of a chromatographic purity of 98,8 %.

**Example 4**

115 g of disodium 1,1-cyclobutanedicarboxylate were dissolved in 600 g of water, and 635 g of an aqueous cis-diamminediaquadinitrateplatinum(II) complex solution (platinum content 80 mg/ml) were added. The mixture was agitated and heated, while exposed to ultrasonic waves of a frequency of 40 kHz, 7 min to 35 °C. Within 5 min, the solution was cooled under agitation in an ultrasonic field to 0 °C whereupon the product was isolated by filtration and washed two times with 50 ml of water. Finally, it was purified by recrystallization under aseptic conditions as described in example 2b.

There were obtained 52 g of micro-crystalline complex I having a chromatographic purity of 99,2 %.

The product was dosed in 1000±50 mg doses into sterile 100 ml bottles closed with a sterile rubber stopper and sealed with an aluminum cap.

There were obtained 50 bottles of the cytostatic agent each containing 1000 mg of complex I in the form of sterile micronized powder which could be easily dissolved, before administration, in 100 ml of sterile 5 % glucose solution.

**Claims**

1. Cytostatic agent on the basis of cis-diammine-1,1-cyclobutanedicarboxylate platinum(II) complex of the general formula I,

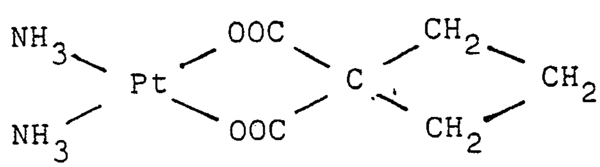

(I),

**characterised** in that

it comprises at least 60 % of particles having a particle size of ≦ 25 µm, at least 95 % of particles having a particle size of ≦ 50 µm and at least 99 % of particles having a particle size of ≦ 75 µm, and having a purity of at least 98 % and a silver content ≦ $1 \cdot 10^{-5}$ %.

2. The cytostatic agent according to claim 1, obtainable by the following steps:
(A) agitating and/or applying sound wave irradiation to a reaction mixture having a pH of 4 to 11 and comprising
- 1 part by mass of a cis-diamminedihalogen platinum(II) complex of general formula II,

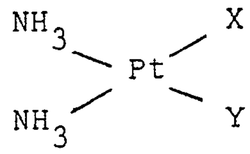 (II),

wherein X and Y are Cl, Br or I
and/or of products of hydrolysis of complex of formula II,

- 1 to 5 parts by mass of 1,1-cyclobutanedi-carboxylic acid and/or salts thereof and/or esters thereof,
- 3 to 30 parts by mass of water, optionally containing a water-miscible organic solvent in an amount of 1 to 10 parts by mass,
- and optionally 0,01 to 2 parts by mass of a sorbent

at a temperature of 20 to 100 °C,
(B) optionally purifying the mixture by addition of 0,01 to 0,5 parts by mass of a sorbent,
(C) filtering the mixture,
(D) cooling down the product to a temperature of -5 to +50 °C,
and
(E) isolating the cytostatic agent.

**3.** A method of preparing the cytostatic agent according to claim 1 or 2,
characterised by the following steps:
(A) agitating and/or applying sound wave irradiation to a reaction mixture having a pH of 4 to 11 and comprising

- 1 part by mass of a cis-diamminedihalogen platinum(II) complex of general formula II,

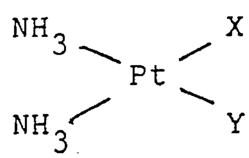 (II),

wherein X and Y are Cl, Br oder I
and/or of products of hydrolysis of complex of formula II,

- 1 to 5 parts by mass of 1,1-cyclobutanedicarboxylic acid and/or salts thereof and/or esters thereof,
- 3 to 30 parts by mass of water, optionally containing a water-miscible organic solvent in an amount of 1 to 10 parts by mass,
- and optionally 0,01 to 2 parts by mass of a sorbent

at a temperature of 20 to 100 °C,
(B) optionally purifying the mixture by addition of 0,01 to 0,5 parts by mass of a sorbent,
(C) filtering the mixture,
(D) cooling down the product to a temperature of -5 to +50 °C,
and
(E) isolating the cytostatic agent.

**4.** The method according to claim 3, characterised in that ethanol, isopropanol and/or glycerol are used as organic solvents in step A.

**5.** The method according to claim 3 or 4, characterised in that sound waves of a frequency of 15 to 50 kHz are applied in one or more of steps A to D.

**6.** The method according to claim 5, characterised in that sound wave irradiation is applied for a period of 1 to 300 min.

**7.** The method according to one of claims 3 to 6, characterised in that activated carbon and/or an ion-exchanger are used as sorbent in steps A and/or B.

8. The method according to one of claims 3 to 7, characterised in that cooling down in step D is carried out at a cooling rate of 2 to 25 °C/min.

9. The method according to one of claims 3 to 8, characterised in that agitation is applied in steps A and/or D.

10. The method according to one of claims 3 to 9, characterised in that sound waves of a frequency of 15 to 20 kHz are applied in steps A and/or D.

11. The method according to one of claims 3 to 10, characterised in that the product obtained in step E is washed, dried and sorted or classified.

12. The method according to one of claims 3 to 11, characterised in that the platinum complex of formula I is treated by dissolving one part by mass thereof in 5 to 25 parts by mass of water, optionally containing 0,001 to 0,1 part by mass of 1,1-cyclobutanedicarboxylic acid and/or a salt thereof, at a temperature of from 70 to 95 °C, or, optionally, by adding 0,002 to 0,5 parts by mass of the sorbent, filtering and cooling the solution to a temperature of -5 to +50 °C at a rate of 2 to 25 °C/min under agitation and, preferably, exposure to sound waves of a frequency of preferably 15 to 20 kHz, whereupon the micro-crystalline product is isolated, washed and, optionally, dried and sorted.

13. The method according to one of claims 3 to 12, characterised in that the product obtained in step E is washed with water and/or dimethylformamide and/or dimethylacetamide.

14. Pharmacological compositions, characterised in that they comprise the cytostatic agent according to claim 1 or 2 and pharmacologically admissible adjuvants and/or carriers.

15. Pharmacological compositions according to claim 14, characterised in that they comprise the sterile, dry micro-crystalline powder of the cytostatic agent and saccharides and/or organic acids and/or hydroxy acids and/or salts thereof and/or alkali metal and/or alkaline earth metal chlorides.

16. Pharmacological compositions according to claim 14 or 15, characterised by 1 part by mass of the cytostatic agent and 0,1 to 10 parts by mass of lactose and/or glucose and/or saccharose and/or citric acid and/or ascorbic acid and/or salts thereof, and/or sodium chloride and/or potassium chloride and/or magnesium chloride and/or calcium chloride.

17. Pharmacological compositions according to claim 16 for injection, characterised in that they are present in the form of an aqueous solution in 100 to 2000 parts by mass of water and having a pH of 2 to 7.

## Revendications

1. Agent cytostatique à base du complexe du platine(II) cis-diammine-1,1-cyclobutanedicarboxylate de formule générale I

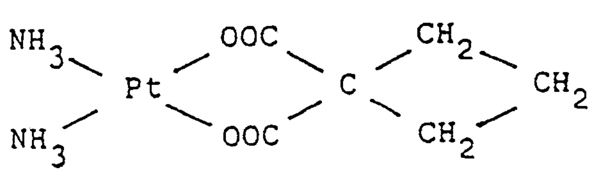

(I),

caractérisé en ce qu'il comprend au moins 60 % de particules d'une grosseur ≦ 25 μm, au moins 95 % de particules d'une grosseur ≦ 50 μm et au moins 99 % de particules d'une grosseur ≦ 75 μm, et a une pureté d'au moins 98 % et une teneur en argent ≦ $1.10^{-5}$ %.

2. Agent cytostatique selon la revendication 1, pouvant être obtenu par les étapes suivantes :
(A) agitation et/ou application d'une irradiation par ondes sonores à un mélange réactionnel ayant un

pH compris entre 4 et 11, et comprenant :

- 1 partie en poids d'un complexe du platine(II) cis-diamminedihalogène de formule générale II

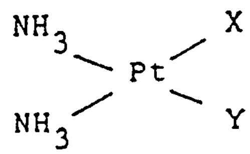

(II),

dans laquelle X et Y représentent Cl, Br ou I ;
et/ou de produits d'hydrolyse du complexe de formule II,
- 1 à 5 parties en poids d'acide 1,1-cyclobutanedicarboxylique et/ou des sels de celui-ci et/ou des esters de celui-ci,
- 3 à 30 parties en poids d'eau, contenant éventuellement un solvant organique miscible avec l'eau, à raison de 1 à 10 parties en poids,
- et éventuellement 0,01 à 2 parties en poids d'un sorbant,

à une température comprise entre 20 et 100 °C,
(B) éventuellement, purification du mélange par addition de 0,01 à 0,5 partie en poids d'un sorbant,
(C) filtration du mélange,
(D) refroidissement du produit à une température comprise entre -5 et +50 °C, et
(E) isolement de l'agent cytostatique.

3. Procédé de préparation de l'agent cytostatique selon la revendication 1 ou 2, caractérisé en ce qu'il comprend les étapes suivantes :
(A) agitation et/ou application d'une irradiation par ondes sonores à un mélange réactionnel ayant un pH compris entre 4 et 11, et comprenant :
- 1 partie en poids d'un complexe du platine(II) cis-diamminedihalogène de formule générale II

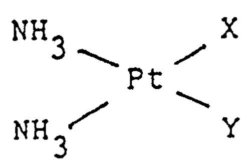

(II),

dans laquelle X et Y représentent Cl, Br ou I :
et/ou de produits d'hydrolyse du complexe de formule II,
- 1 à 5 parties en poids d'acide 1,1-cyclobutanedicarboxylique et/ou des sels de celui-ci et/ou des esters de celui-ci,
- 3 à 30 parties en poids d'eau, contenant éventuellement un solvant organique miscible avec l'eau, à raison de 1 à 10 parties en poids,
- et éventuellement 0,01 à 2 parties en poids d'un sorbant,

à une température comprise entre 20 et 100 °C,
(B) éventuellement, purification du mélange par addition de 0,01 à 0,5 partie en poids d'un sorbant,
(C) filtration du mélange,
(D) refroidissement du produit à une température comprise entre -5 et +50 °C, et
(E) isolement de l'agent cytostatique.

4. Procédé selon la revendication 3, caractérisé en ce qu'on utilise de l'éthanol, de l'isopropanol et/ou du glycérol, comme solvants organiques, dans l'étape A.

5. Procédé selon la revendication 3 ou 4, caractérisé en ce qu'on applique des ondes sonores d'une fréquence de 15 à 50 kHz dans une ou plusieurs des étapes A à D.

6. Procédé selon la revendication 5, caractérisé en ce qu'on applique une irradiation par ondes sonores pendant une durée de 1 à 300 minutes.

7. Procédé selon l'une des revendications 3 à 6, caractérisé en ce qu on utilise du charbon actif et/ou un

échangeur d'ions, comme sorbant, dans les étapes A et/ou B

**8.** Procédé selon l'une des revendications 3 à 7, caractérisé en ce qu'on réalise le refroidissement de l'étape D à une vitesse de refroidissement de 2 à 25° C/minute.

**9.** Procédé selon l'une des revendications 3 à 8, caractérisé en ce qu on applique une agitation dans les étapes A et/ou D.

**10.** Procédé selon l'une des revendications 3 à 9, caractérisé en ce qu'on applique des ondes sonores d'une fréquence de 15 à 20 kHz dans les étapes A et/ou D.

**11.** Procédé selon l'une des revendications 3 à 10, caractérisé en ce que le produit obtenu dans la phase E est lavé, séché, et trié ou classé.

**12.** Procédé selon l'une des revendications 3 à 11, caractérisé en ce qu'on traite le complexe du platine de formule I en ce qu'on dissout 1 partie en poids de celui-ci dans 5 à 25 parties d'eau, contenant éventuellement 0,001 à 0,1 partie en poids d'acide 1,1-cyclobutanedicarboxylique et/ou un sel de celui-ci, à une température comprise entre 70 et 95° C ou, éventuellement, on ajoute 0,002 à 0,5 partie en poids du sorbant, on filtre et refroidit la solution à une température comprise entre -5 et +50° C, à une vitesse de 2 à 25° C/minute, sous agitation, et, de préférence, on la soumet à des ondes sonores d'une fréquence comprise de préférence entre 15 et 20 kHz, après quoi on isole le produit microcristallin, on le lave et, éventuellement, on le sèche et trie.

**13.** Procédé selon l'une des revendications 3 à 12, caractérisé en ce qu'on lave le produit obtenu dans l'étape E avec de l'eau et/ou du diméthylformamide et/ou du diméthylacétamide.

**14.** Compositions pharmacologiques, caractérisées en ce qu'elles comprennent l'agent cytostatique selon la revendication 1 ou 2 et des adjuvants et/ou supports pharmacologiquement acceptables.

**15.** Compositions pharmacologiques selon la revendication 14, caractérisées en ce qu'elles comprennent la poudre microcristalline, stérile et sèche, de l'agent cytostatique et des saccharides et/ou des acides organiques et/ou des acides hydroxyliques et/ou des sels de ceux-ci et/ou des chlorures de métaux alcalins et/ou de métaux alcalinoterreux.

**16.** Compositions pharmacologiques selon la revendication 14 ou 15, caractérisées en ce qu'elles contiennent 1 partie en poids de l'agent cytostatique et 0,1 à 10 parties en poids de lactose et/ou de glucose et/ou de saccharose et/ou d'acide citrique et/ou d'acide ascorbique et/ou des sels de ceux-ci, et/ou du chlorure de sodium et/ou du chlorure de potassium et/ou du chlorure de magnésium et/ou du chlorure de calcium.

**17.** Compositions pharmacologiques selon la revendication 16, destinées à être injectées, caractérisées en ce qu'elles se présentent sous la forme d'une solution aqueuse dans 100 à 2.000 parties en poids d'eau et ayant un pH compris entre 2 et 7.

## Ansprüche

**1.** Cytostatisches Mittel auf der Basis des Platin(II)-cis-diammin-1,1-cyclobutandicarboxylat-Komplexes der allgemeinen Formel I,

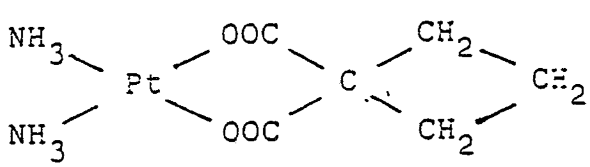

(I),

dadurch gekennzeichnet, daß es

mindestens 60 % Partikel einer Teilchengröße ≦ 25 μm,
mindestens 95 % Partikel einer Teilchengröße ≦ 50 μm und
mindestens 99 % Partikel einer Teilchengröße ≦ 75 μm
aufweist und eine Reinheit von mindestens 98 % und
einen Silbergehalt ≦ $1 \cdot 10^{-5}$ % besitzt.

**2.** Cytostatisches Mittel nach Anspruch 1, erhältlich durch folgende Verfahrensschritte:
(A) Rühren und/oder Beschallen eines Reaktionsgemischs mit Schallwellen, das einen pH-Wert von 4 bis 11 aufweist und enthält:
- 1 Masseteil eines Platin(II)-cis-diammindi-halogen-Komplexes der allgemeinen Formel II,

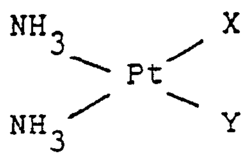 (II),

in der X und Y Cl, Br oder I bedeuten,
und/oder von Hydrolyseprodukten des Komplexes der Formel II,
- 1 bis 5 Masseteile 1,1-Cyclobutandicarbonsäure und/oder von Salzen davon und/oder von Estern davon,
- 3 bis 30 Masseteile Wasser, das wahlweise ein mit Wasser mischbares organisches Lösungsmittel in einer Menge von 1 bis 10 Masseteilen enthält,
- sowie wahlweise 0,01 bis 2 Masseteile eines Sorbens

bei einer Temperatur von 20 bis 100 °C,
(B) wahlweise Reinigen des Gemischs durch Zusatz von 0,01 bis 0,5 Masseteilen eines Sorbens,
(C) Filtrieren des Gemischs,
(D) Abkühlen des Produkts auf eine Temperatur von -5 bis +50 °C
und
(E) Isolieren des cytostatischen Mittels.

**3.** Verfahren zur Herstellung des cytostatischen Mittels nach Anspruch 1 oder 2,

gekennzeichnet durch folgende Verfahrensschritte:
(A) Rühren und/oder Beschallen eines Reaktionsgemischs mit Schallwellen, das einen pH-Wert von 4 bis 11 aufweist und enthält:
- 1 Masseteil eines Platin(II)-cis-diammindi-halogen-Komplexes der allgemeinen Formel II,

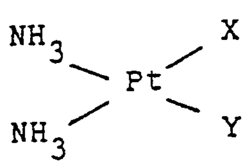 (II),

in der X und Y Cl, Br oder I bedeuten,
und/oder von Hydrolyseprodukten des Komplexes der Formel II,
- 1 bis 5 Masseteile 1,1-Cyclobutandicarbonsäure und/oder von Salzen davon und/oder von Estern davon,
- 3 bis 30 Masseteile Wasser, das wahlweise ein mit Wasser mischbares organisches Lösungsmittel in einer Menge von 1 bis 10 Masseteilen enthält,
- sowie wahlweise 0,01 bis 2 Masseteile eines Sorbens

bei einer Temperatur von 20 bis 100 °C,
(B) wahlweise Reinigen des Gemischs durch Zusatz von 0,01 bis 0,5 Masseteilen eines Sorbens,
(C) Filtrieren des Gemischs,
(D) Abkühlen des Produkts auf eine Temperatur von -5 bis +50 °C
und

(E) Isolieren des cytostatischen Mittels.

4. Verfahren nach Anspruch 3, dadurch gekennzeichnet, daß als organische Lösungsmittel in Schritt A Ethanol, Isopropanol und/oder Glycerin verwendet werden.

5. Verfahren nach Anspruch 3 oder 4, dadurch gekennzeichnet, daß in einem oder mehreren der Schritte A bis D mit Schallwellen eine Frequenz von 15 bis 50 kHz beschallt wird.

6. Verfahren nach Anspruch 5, dadurch gekennzeichnet, daß die Beschallung mit Schallwellen während einer Zeitdauer von 1 bis 300 min durchgeführt wird.

7. Verfahren nach einem der Ansprüche 3 bis 6, dadurch gekennzeichnet, daß als Sorbentien in den Schritten A und/oder B Aktivkohle und/oder ein Ionenaustauscher verwendet werden.

8. Verfahren nach einem der Ansprüche 3 bis 7, dadurch gekennzeichnet, daß die Abkühlung in Schritt D mit einer Abkühlgeschwindigkeit von 2 bis 25 °C/min durchgeführt wird.

9. Verfahren nach einem der Ansprüche 3 bis 8, dadurch gekennzeichnet, daß in den Schritten A und/oder D gerührt wird.

10. Verfahren nach einem der Ansprüche 3 bis 9, dadurch gekennzeichnet, daß in den Schritten A und/oder D mit Schallwellen einer Frequenz von 15 bis 20 kHz beschallt wird.

11. Verfahren nach einem der Ansprüche 3 bis 10, dadurch gekennzeichnet, daß das in Schritt F erhaltene Produkt gewaschen, getrocknet und sortiert oder klassiert wird.

12. Verfahren nach einem der Ansprüche 3 bis 11, dadurch gekennzeichnet, daß der Platinkomplex der Formel I behandelt wird durch Lösen von 1 Masseteil in 5 bis 25 Masseteilen Wasser, das wahlweise 0,001 bis 0,1 Masseteil 1,1-Cyclobutandicarbonsäure und/oder eines Salzes davon enthält, bei einer Temperatur von 70 bis 95 °C oder wahlweise durch Zusatz von 0,002 bis 0,5 Masseteilen Sorbens, Filtrieren und Abkühlen der Lösung auf eine Temperatur von -5 bis +50 °C mit einer Abkühlgeschwindigkeit von 2 bis 25 °C/min unter Rühren und, vorzugsweise, Beschallen mit Schallwellen einer Frequenz von vorzugsweise 15 bis 20 kHz, worauf das mikrokristalline Produkt isoliert, gewaschen sowie wahlweise getrocknet und sortiert wird.

13. Verfahren nach einem der Ansprüche 3 bis 12, dadurch gekennzeichnet, daß das in Schritt E erhaltene Produkt mit Wasser und/oder Dimethylformamid und/oder Dimethylacetamid gewaschen wird.

14. Pharmakologische Zusammensetzungen, dadurch gekennzeichnet, daß sie das cytostatische Mittel nach Anspruch 1 oder 2 und pharmakologisch zulässige Hilfsstoffe und/oder Trägerstoffe enthalten.

15. Pharmakologische Zusammensetzungen nach Anspruch 14, dadurch gekennzeichnet, daß sie das sterile, trockene mikrokristalline Pulver des cytostatischen Mittels sowie Saccharide und/oder organische Säuren und/oder Hydroxysäuren und/oder Salze davon und/oder Alkalimetall-und/oder Erdalkalimetallchloride enthalten.

16. Pharmakologische Zusammensetzungen nach Anspruch 14 oder 15, gekennzeichnet durch 1 Masseteil des cytostatischen Mittels und 0,1 bis 10 Masseteile Lactose und/oder Glucose und/oder Saccharose und/oder Citronensäure und/oder Ascorbinsäure und/oder Salze davon und/oder Natriumchlorid und/oder Kaliumchlorid und/oder Magnesiumchlorid und/oder Calciumchlorid.

17. Pharmakologische Zusammensetzungen nach Anspruch 16 zur Injektion, dadurch gekennzeichnet, daß sie in Form einer wäßrigen Lösung in 100 bis 2000 Masseteilen Wasser vorliegen und einen pH-Wert von 2 bis 7 aufweisen.